(19) Europäisches Patentamt / European Patent Office / Office européen des brevets

(11) **EP 1 731 143 B1**

(12) **EUROPEAN PATENT SPECIFICATION**

(45) Date of publication and mention
of the grant of the patent:
**26.11.2008 Bulletin 2008/48**

(51) Int Cl.:
***A61K 9/70*** *(2006.01)*        ***A61K 31/135*** *(2006.01)*
***A61K 47/02*** *(2006.01)*

(21) Application number: **06011513.6**

(22) Date of filing: **02.06.2006**

(54) **Percutaneous absorption-type pharmaceutical preparation using a metal chloride, preferably sodium chloride, for preventing cohesive failure**

Pharmazeutische Zusammensetzung zur perkutane Absorption unter Verwendung von einem Metalchlorid, vorzugsweise Natriumchlorid, zur Vorbeugung von Kohäsionsschwäche

Préparation pharmaceutique pour absorption percutanée employant un chloride de métal, préférablement le chloride de sodium, pour empêcher la défaillance de cohésion

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.06.2005 JP 2005165051**

(43) Date of publication of application:
**13.12.2006 Bulletin 2006/50**

(73) Proprietors:
• **NITTO DENKO CORPORATION**
  **Osaka (JP)**
• **Fujimoto Co., Ltd.**
  **Matsubara-shi,**
  **Osaka 580-0011 (JP)**

(72) Inventors:
• **Inosaka, Keigo**
  **Ibaraki-shi**
  **Osaka (JP)**
• **Muraoka, Takateru**
  **Ibaraki-shi**
  **Osaka (JP)**
• **Iwao, Yoshihiro**
  **Ibaraki-shi**
  **Osaka (JP)**
• **Takada, Akio**
  **Ibaraki-shi**
  **Osaka (JP)**
• **Tsuda, Toshinobu**
  **Ibaraki-shi**
  **Osaka (JP)**
• **Sekiya, Junichi**
  **Ibaraki-shi**
  **Osaka (JP)**

(74) Representative: **Grünecker, Kinkeldey, Stockmair & Schwanhäusser Anwaltssozietät Leopoldstrasse 4 80802 München (DE)**

(56) References cited:
WO-A2-01/43775        WO-A2-96/40085
US-A- 5 254 338        US-A- 5 462 746
US-A- 5 480 649        US-A- 5 645 855
US-A- 6 121 508        US-A1- 2001 006 628
US-A1- 2004 062 759

## Description

### Field of the Invention

The present invention relates to a percutaneous absorption-type pharmaceutical preparation containing (-)-(R)-N,α-dimethyl-N-2-propynylphenethylamine (hereinafter referred to as selegiline) and/or its hydrochloride, (-)-(R)-N,α-dimethyl-N-2-propynylphenethylamine monohydrochloride (hereinafter referred to as selegiline hydrochloride). Concretely, it is a percutaneous absorption-type pharmaceutical preparation that is applied to the skin of a living body so as to continuously administer the drug into the body through the skin.

### Background of the Invention

Selegiline or its hydrochloride, selegiline hydrochloride, which is a basic drug, is a remedy for Parkinson's disease, and is known as a monoamine oxidase (MAO) inhibitor. MAO includes different subtypes of A-type (MOA-A) and B-type (MAO-B), and selegiline hydrochloride is a B-type selective inhibitor. On the other hand, regarding the administration method of selegiline hydrochloride, it has heretofore been reported that oral administration of a large amount of selegiline hydrochloride inhibits MAO-A, thereby exhibiting an antidepressant effect. MAO-A exists much in digestive tracts, and inhibiting it results in cataplectic hypertension, and therefore an administration mode not too much delivering the drug to digestive tracts has been desired.

As opposed to it, percutaneous absorption-type pharmaceutical preparations may evade drug absorption by digestive tubes and may evade initial drug passing through liver, and therefore they are expected as excellent administrationmodes in administration of such basic drubs. Regarding their types, various types such as reservoir-type or matrix-type of percutaneous absorption-type pharmaceutical preparations are known. In general consideration of drug administration for diseases, the administration period is preferably shorter, but in real life, drugs are often administered for a long period of time. In case where a percutaneous absorption-type pharmaceutical preparation is applied during such a long-term administration period, then the administration shall be repeated almost everyday. The site to which the percutaneous absorption-type pharmaceutical preparation is applied is preferably one except the moving sites of a body, and it is understood that the pharmaceutical preparation is limited in point of the applicable site thereof.

On the assumption of repeated application thereof from the above, the percutaneous absorption-type pharmaceutical preparation is required to be as soft as possible to the skin so as not to irritate the skin surface to cause keratin damage, or that is, the preparation is desired to be less irritative. Regarding it, there may be mentioned a method of changing the composition of the adhesive itself to be employed so as to suitably lower the adhesive power thereof to the skin, or a method of making the adhesive layer gel by adding a liquid ingredient thereto so that the adhesive layer may have a soft touch. For the gel formation, a method has heretofore been employed, which comprises adding a crosslinking agent to an adhesive to increase the cohesive force thereof so that the adhesive layer may hold a liquid ingredient miscible therein.

Regarding the gel formation, however, when a basic drug is used in a percutaneous absorption-type pharmaceutical preparation, then it may react with a polyfunctional isocyanate or the like compound used as a crosslinking agent therein, and the crosslinking agent could not sufficiently exhibit its function. In such a case, it is known that a metal chelate or the like crosslinking agent may act predominantly, therefore exhibiting its effect.

Recently, however, it has been reported that when a percutaneous absorption-type pharmaceutical preparation that comprises a gel of a combination of a basic drug and a metal chelate is applied to humans, then the crosslinked sites in the adhesive layer may be broken by lactic acid, a minor component of sweat perspiring through sweat glands, therefore causing cohesive failure in stripping the pharmaceutical preparation.

Regarding it, methods have been proposed for evading the problem; one comprising adding another component of polyalcohol so as to more conveniently exhibit the intrinsic effect of the metal chelate (Patent Reference 1: JP-A 2003-62058), and the other comprising planning a placebo layer that comprises a crosslinking agent not influenced by lactic acid for the adhesive layer to be in direct contact to the skin followed by superposing, as an upper layer thereon, an adhesive layer that contains a basic drug and is crosslinked with a metal chelate (Patent Reference 2: JP-A 2004-10525).

However, in the former method (Patent Reference 1), since the polyalcohol is a hydrophilic compound, it may uniformly dissolve in the adhesive layer that is in a hydrophobic environment, in an amount of at most about 5 % by weight or so, and when its amount exceeds the limit, then there may occur a problem of its blooming from the adhesive layer. Accordingly, it has been found that, when a relatively large amount of a basic drug is incorporated in the adhesive layer, then the amount of the polyalcohol that may uniformly dissolve in the adhesive layer may further decrease and therefore the cohesive failure of the adhesive layer could not be sufficiently prevented. Increasing the amount of the polyalcohol to be in the adhesive layer may be taken into consideration, by which, however, the adhesive content of the adhesive layer is lowered therefore reducing the adhesive force of the layer.

Regarding the latter method (Patent Reference 2), the cohesion failure on stripping, which is caused by the penetration and diffusion of lactic acid into the skin through the attached surface of the preparation, could be prevented, but in fact, since the sides of the percutaneous absorption-type pharmaceutical preparation are in contact with the skin in its application to the skin, it has been found that the cohesion failure at the edges of the preparation owing to the penetration of lactic acid trough the sides thereof could not be evaded.

## SUMMARY OF THE INVENTION

An object of the invention is to provide a stable percutaneous absorption-type pharmaceutical preparation for percutaneous administration of selegiline and/or selegiline hydrochloride, which does not suffer a decrease in the cohesive force of the adhesive layer therein even in the presence of sweat components due to perspiration during wear and which is free from cohesive failure and resultant adhesive remaining when stripped off.

The present inventors made intensive investigations in order to accomplish the object. As a result, they have found that, when an inorganic metal compound such as sodium chloride is made to coexist in a percutaneous absorption-type pharmaceutical preparation containing selegiline and/or selegiline hydrochloride, then a stable pharmaceutical preparation can be obtained which does not cause a decrease in preparation can be obtained which does not cause a decrease in the cohesive force of the adhesive layer even when the lactic acid in sweat is taken into the preparation and which is free from adhesive remaining when stripped off. Thus, the present invention has been completed.

The invention provides the following.

(1) A percutaneous absorption-type pharmaceutical preparation which comprises: a support; and an adhesive layer containing a metal chloride, an adhesive and at least one of (-)-(R)-N,α-dimethyl-N-2-propynylphenethylamine and its hydrochloride, wherein the adhesive layer is subjected to a crosslinking treatment.
(2) The percutaneous absorption-type pharmaceutical preparation of above (1), wherein the crosslinking treatment is performed by a metal chelate compound.
(3) The percutaneous absorption-type pharmaceutical preparation of above (1) or (2), wherein the adhesive contains an acrylic polymer adhesive.
(4) The percutaneous absorption-type pharmaceutical preparation of any one of above (1) to (3), wherein the adhesive layer contains a liquid plasticizer.
(5) The percutaneous absorption-type pharmaceutical preparation of above (4), wherein the liquid plasticizer is a fatty acid ester of a higher fatty acid having from 12 to 16 carbon atoms and a lower monoalcohol having from 1 to 4 carbon atoms.

The percutaneous absorption-type pharmaceutical preparation of the invention can prevent the reduction in the cohesive force of the adhesive layer therein to be caused by infiltration of lactic acid, a sweat component, into it. Accordingly, the invention provides a stable absorption-type pharmaceutical preparation, in which the content of selegiline and/or selegiline hydrochloride can be set freely, and which does not suffer a decrease in the cohesive force of the adhesive layer therein even in the presence of sweat components due to perspiration during wear and is free from cohesive failure and resultant adhesive remaining when stripped off.

## DETAILED DESCRIPTION OF THE INVENTION

The invention is described in detail hereinunder. The percutaneous absorption-type pharmaceutical preparation of the invention is for percutaneous administration of selegiline and/or selegiline hydrochloride, in which the adhesive layer contains selegiline and/or selegiline hydrochloride and which is used as an antidepressant. Its other applications are for anti-parkinsonism, anti-alzheimerism, anti-epilepsy, prevention of seasickness, remedy of schizophrenia, sustenance and protection of neurocytes, improvement of acetylcholine-based neurotransmission, remedy of glaucoma, antiaging, and remedy of HIV-associated cognitive function failure and ADHD (attention deficit hyperactivity disorder).

The support to be used in the invention is not particularly limited. However, it is preferably made of a material which prevents the liquid plasticizer and selegiline in the preparation from passing through the support and going out from its back side to result in a decrease in their content. Namely, the support is preferably made of a material impermeable to these ingredients. Concretely, it includes films of polyesters, nylons, polyvinyl chlorides, polyethylenes, polypropylenes, ethylene-vinyl acetate copolymers, polytetrafluoroethylenes, ionomer resins; and metal foils, and their laminate films. Of those, laminate films of a poreless film and a porous film of the above-mentioned material are preferred for the support and the adhesive layer is formed on the porous film in order to improve the adhering capability (anchoring capability) of the support to the adhesive layer.

The porous film is not particularly limited so far as its anchoring capability for the adhesive layer to be thereon is good. For example, it includes paper, woven fabrics, nonwoven fabrics, mechanically-perforated sheets. In particular, paper, woven fabrics and nonwoven fabrics are preferred. The thickness of the porous film may be generally from 10 to 500 $\mu$m in consideration of improving the anchoring capability of the film and of the flexibility of the percutaneous absorption-type pharmaceutical preparation. For thin percutaneous absorption-type pharmaceutical preparations such as plaster-type or adhesive tape-type ones, the thickness of the porous film may be generally from 10 to 200 $\mu$m or so. When the porous film is formed of a woven fabric or nonwoven fabric, then its basis weight may be preferably from 5 to 30 g/m$^2$ for improving the anchoring capability of the film.

The adhesive in the adhesive layer to be formed on at least one side of the support is not also particularly limited. Preferably, it is formed of a copolymer prepared through copolymerization of an alkyl (meth)acrylate as the essential ingredient thereof (that is, "acrylic adhesive"). Preferably, the alkyl group of the alkyl (meth) acrylate has at least 4 carbon atoms, and may be linear or branched. Concretely, it includes butyl, pentyl, hexyl, heptyl, octyl, 2-ethylhexyl, nonyl, decyl, undecyl, dodecyl, tridecyl. One or more such alkyl (meth) acrylates may be used herein either singly or as combined.

The monomer capable of copolymerizing with the alkyl (meth) acrylate includes, for example, carboxyl group-having monomers and anhydride thereof such as (meth)acrylic acid, itaconic acid, maleic acid, maleic anhydride; sulfonic acid monomers such as styrenesulfonic acid, allylsulfonic acid, sulfopropyl (meth)acrylate, (meth) acryloyloxynaphthalenesulfonic acid, acrylamidomethylsulfonic acid; hydroxyl group-having monomers such as hydroxypropyl (meth)acrylate; amido group-having (meth)acrylic acid derivatives such as (meth)acxylamide, dimethyl(meth)acrylamide, N-butyl (meth)acrylamide, N-methylol (meth) acrylamide; aminoalkyl (meth) acrylates such as aminoethyl (meth) acrylate, dimethylaminoethyl (meth) acrylate, t-butylaminoethyl (meth)acrylate; alkoxy (meth) acrylates such as methoxyethyl (meth)acrylate, ethoxyethyl (meth)acrylate, tetrahydrofurfuryl (meth)acrylate; alkoxyalkylene glycol (meth) acrylates such as methoxyethylene glycol (meth) acrylate, methoxydiethylene glycol (meth)acrylate, methoxypolyethylene glycol (meth) acrylate, methoxypolypropylene glycol (meth)acrylate; (meth)acrylonitrile; vinyl-having compounds such as vinyl acetate, vinyl propionate, methylvinylpyrrolidone, vinylpyridine, vinylpiperidone, vinylpyrimidine, vinylpiperazine, vinylpyrrole, vinylimidazole, vinylcaprolactam, vinyloxazole, vinylmorpholine. One or more of these may be used herein either singly or as combined.

Especially preferred copolymers for use herein are, for example, copolymers of 2-ethylhexyl acrylate, N-vinyl-2-pyrrolidone and acrylic acid; and copolymers of 2-ethylhexyl acrylate, 2-hydroxyethyl acrylate and vinyl acetate.

Though varying depending on the copolymerization composition thereof, the glass transition temperature of the acrylic adhesive maybe, in general, preferably from-60 to -10°C, more preferably from -43 to -27°C.

The adhesive layer in the invention may contain a rubber-based adhesive, a silicone-based adhesive, a vinyl ester-based adhesive and the like, in addition to the acrylic adhesive.

A liquid plasticizer may be added to the adhesive layer.

The liquid plasticizer is not particularly limited as long as it is liquid by itself at room temperature and has a plasticizing effect and is compatible with the adhesive to be used (e.g., adhesive polymer). Preferably, it may improve the percutaneous absorbability and the storage stability of selegiline. It may be incorporated into the pharmaceutical preparation for the purpose of further increasing the drug solubility in the adhesive layer.

Concretely, the liquid plasticizer includes fatty acid esters of a higher fatty acid having from 12 to 16 carbon atoms and a lower monoalcohol having from 1 to 4 carbon atoms; fatty acids having from 8 to 10 carbon atoms [e.g., caprylic acid (octanoic acid, C8), pelargonic acid (nonanoic acid, C9), capric acid (decanoic acid, C10)]; glycols such as ethylene glycol, diethylene glycol, triethylene glycol, polyethylene glycol, propylene glycol, polypropylene glycol; oils and fats such as olive oil, castor oil, squalane, lanolin; organic solvents such as ethyl acetate, ethyl alcohol, dimethyldecyl sulfoxide, methyloctyl sulfoxide, dimethyl sulfoxide, dimethylformamide, dimethylacetamide, dimethyllaurylamide, dodecylpyrrolidone, isosorbitol; liquid surfactants; known plasticizers such as diisopropyl adipate, phthalates, diethyl sebacate; hydrocarbons such as liquid paraffin; and others such as ethoxylated stearyl alcohol, glycerin esters (that are liquid at room temperature), isotridecyl myristate, N-methylpyrrolidone, ethyl oleate, oleic acid, diisopropyl adipate, diisopropyl palmitate, octyl palmitate, 1,3-propanediol, glycerin. Preferred are fatty acid esters of a higher fatty acid having from 12 to 16 carbon atoms and a lower monoalcohol having from 1 to 4 carbon atoms.

The higher fatty acid having from 12 to 16 carbon atoms in the fatty acid esters includes saturated and unsaturated fatty acids, but saturated fatty acids are preferred. The lower monoalcohol having from 1 to 4 carbon atoms may be linear or branched. Preferred examples of the higher fatty acid having from 12 to 16 carbon atoms are lauric acid (C12), myristic acid (C14) and palmitic acid (C16); and preferred examples of the lower monoalcohol having from 1 to 4 carbon atoms are isopropyl alcohol, ethyl alcohol, methyl alcohol and propyl alcohol. Of those, isopropyl myristate is a preferred fatty acid ester.

One or more such liquid plasticizers may be used herein either singly or as combined. In addition, in consideration of improving the percutaneous absorbability of selegiline, the fatty acid ester may be combined with a fatty acid having from 8 to 10 carbon atoms and/or glycerin for the liquid plasticizer.

The amount of the liquid plasticizer to be incorporated is preferably from 10 to 140 parts by weight, more preferably from 40 to 100 parts by weight, per 100 parts by weight of the adhesive polymer. When the amount of the liquid plasticizer incorporated is smaller than 10 parts by weight, then the plasticization of the adhesive layer may be insufficient and the skin irritation of the pharmaceutical preparation could not be reduced. On the contrary, when the amount thereof is larger than 140 parts by weight, then the liquid plasticizer could not be held in the adhesive layer even by the cohesive force of the adhesive therein and it may unfavorably bloom out on the surface of the adhesive layer to lower the adhesiveness of the layer.

Not particularly limited, the crosslinking agent to be used for crosslinking the adhesive layer may be any one that is not influenced by selegiline and/or selegiline hydrochloride in the layer to interfere with the formation of crosslinks therein. For example, it includes organometallic compounds (e.g., zirconium and zinc, zinc acetate, zinc ammonium glycinate); metal alcoholates (e.g., tetraethyl titanate, tetraisopropyl titanate, aluminium isopropylate, aluminium butyrate), and metal chelate compounds (e.g., di-i-propoxybis(acetylacetone) titanate, tetraoctylene glycol titanate, aluminium isopropylate, (ethyl acetoacetate)aluminium diisopropylate, aluminium tris(ethylacetoacetate), aluminium tris (acetylacetate)). Metal chelates are preferred for the crosslinking agent to be used herein. One or more such crosslinking agents may be used, either singly or as combined, for the crosslinking treatment.

Though varying depending on the type of the crosslinking agent and the adhesive used, the amount of the crosslinking agent to be incorporated may be generally from 0.1 to 0.6 parts by weight, preferably from 0.15 to 0.4 parts by weight, per 100 parts by weight of the adhesive that is crosslinked with it.

The gel fraction of the percutaneous absorption-type pharmaceutical preparation of the invention is preferably at least 45 %, more preferably at least 55 %, from the viewpoint of the sustainability of the cohesive force of the adhesive therein.

Selegiline and/or selegiline hydrochloride may be in the adhesive layer of the percutaneous absorption-type pharmaceutical preparation of the invention, as dissolved or dispersed in the adhesive layer.

The content of selegiline and/or selegiline hydrochloride in the adhesive layer may be generally from 2 to 30 % by weight, preferably from 5 to 20 % by weight of the total weight of the adhesive layer.

The adhesive layer of the percutaneous absorption-type pharmaceutical preparation of the invention contains a metal chloride. Concretely, for example, the metal chloride includes sodium chloride, aluminium chloride, stannous chloride, ferric chloride. Any one of these or two or more of these may be used herein either singly or as combined. Preferably, sodium chloride is used.

The metal chloride content of the layer may be generally from 0.1 to 20 parts by weight, preferably from 1 to 10 parts by weight, per 100 parts by weight of the adhesive in the layer. When the content is smaller than 0.1 parts by weight, then the effect of inhibiting the influence of lactic acid in sweat on the pharmaceutical preparation may be insufficient; but on the contrary, when the content is larger than 20 parts by weight, then the inhibiting effect may be enough but the metal chloride could not uniformly disperse in the adhesive, therefore often causing a problem of bad appearance of the pharmaceutical preparation.

The thicknesses of the adhesive layer may be generally from 10 to 300 $\mu$m, preferably from 50 to 200 $\mu$m, from the standpoint of the applicability to the skin and the strippability of the pharmaceutical preparation.

If desired, additives may be incorporated into the adhesive layer. Examples thereof include antioxidants, various pigments, various fillers, stabilizers, drug dissolution aids, and drug dissolution inhibitors.

Not particularly limited in point of its production, the percutaneous absorption-type pharmaceutical preparation of the invention may be produced, for example, according to the following production method.

Selegiline and/or selegiline hydrochloride is mixed with stirring with a metal chloride dispersed in a solvent such as ethanol to prepare a drug-containing liquid. In case where selegiline hydrochloride is used as the drug, then selegiline hydrochloride may be mixed with stirring with a metal hydroxide in a solvent for neutralization to form a metal chloride. Further, after selegiline hydrochloride is mixed with stirring with a metal hydroxide in a solvent to form a metal chloride therein, another metal chloride may be added to the resultant drug-containing liquid. The metal hydroxide includes, for example, sodium hydroxide, potassium hydroxide, calcium hydroxide, magnesiumhydroxide. Sodium hydroxide is preferably used.

The drug-containing liquid is dissolved or dispersed in a solvent or dispersant, for example, along with an adhesive (e.g., acrylic copolymer adhesive), a crosslinking agent and optionally a liquid plasticizer and other additives therein. Not particularly limited, the solvent or the dispersant to be used in forming the adhesive layer may be any ordinary one generally used as a solvent or the like for adhesives, and may be selected in consideration of the type of the adhesive used and the reactivity thereof with the drug. For example, it includes ethyl acetate, toluene, hexane, 2-propanol, methanol, ethanol.

Next, the resultant solution or dispersion is applied onto one side of a support or onto the lubricant-processed side of a release sheet, and dried to form an adhesive layer thereon, and then, this is stuck to a release sheet or a support. Not particularly limited, the release sheet may be any one capable of being readily stripped from the adhesive layer in use. For example, for it, use may be made of a film of a polyester, polyvinyl chloride, polyvinylidene chloride, polyethylene terephthalate or the like in which the side to be in contact with the adhesive layer has been treated with a silicone, or of a laminated film obtained by laminating a polyolefin to wood-free paper or glassine paper. The thickness of the release sheet may be generally 200 μm or smaller, preferably from 25 to 100 μm. Such a release sheet is stuck to the adhesive layer and aged generally at 70°C for 24 to 48 hours to promote the crosslinking in the layer, thereby producing the percutaneous absorption-type pharmaceutical preparation of the invention.

Apart from the above, another production method may also be employed herein, which comprises dissolving or dispersing selegiline and/or selegiline hydrochloride in a solvent or dispersant along with an adhesive (e.g., acrylic copolymer adhesive), a crosslinking agent and optionally a liquid plasticizer and other additives therein to prepare a drug-containing liquid, then adding a metal chloride to the resultant liquid with stirring, applying it onto one side of a support or onto the lubricant-processed side of a release sheet, drying it to form an adhesive layer thereon, and thereafter sticking it to a release sheet or a support.

The shape of the percutaneous absorption-type pharmaceutical preparation of the invention is not particularly limited. Examples thereof include tape forms and sheet forms.

The dose of the percutaneous absorption-type pharmaceutical preparation of the invention varies depending on the kind of the drug used, the age, body weight, and condition of the patient, etc. Usually, however, the dose for an adult is such that the pharmaceutical preparation containing from 5 to 100 mg of selegiline and/or selegiline hydrochloride is applied to a skin area of from 5 to 100 cm$^2$, about once per day or once per 2 days.

Examples

The invention will be described below in more detail with reference to the following Examples, but the invention should not be construed as being limited by these in any way. In the following description, all parts and percents are by weight.

(Preparation of Acrylic Copolymer Adhesive A)

In an inert gas atmosphere, 75 parts of 2-ethylhexyl acrylate, 22 parts of N-vinyl-2-pyrrolidone, 3 parts of acrylic acid and 0.2 parts of azobisisobutyronitrile were subjected to solution polymerization in ethyl acetate at 60°C to prepare a solution of an acrylic copolymer A.

(Preparation of Acrylic Copolymer Adhesive B)

In an inert gas atmosphere, 95 parts of 2-ethylhexyl acrylate, 5 parts of acrylic acid and 0.2 parts of benzoyl peroxide were subjected to solution polymerization in ethyl acetate at 60°C to prepare a solution of an acrylic copolymer B.

<Percutaneous Absorption-type Pharmaceutical Preparations Produced Using Selegiline>

EXAMPLE 1:

49 parts of the acrylic adhesive A, 40 parts of isopropyl myristate and 10 parts of selegiline were mixed and stirred in a container to give a uniform mixture. Next, 1 part of sodium chloride dispersed in ethanol was added to the resultant acrylic adhesive A solution, and stirred. 0.3 parts (relative to the adhesive solid content) of (ethyl acetoacetate) aluminium diisopropylate was added to it, and the viscosity of the resultant mixture was controlled with ethyl acetate added thereto, and this was applied to a polyester film (75 μm thick) so that its dry thickness thereon could be 80 μm, and dried. This was stuck to a polyester film (12 μm thick), and aged at 70°c for 48 hours to obtain a selegiline-containing percutaneous absorption-type pharmaceutical preparation.

EXAMPLE 2:

47 parts of the acrylic adhesive A, 40 parts of isopropyl myristate and 10 parts of selegiline were mixed and stirred in a container to give a uniform mixture. Next, 3 parts of sodium chloride dispersed in ethanol was added to the resultant acrylic adhesive A solution, and stirred. 0.3 parts (relative to the adhesive solid content) of (ethyl acetoacetate) aluminium diisopropylatewas added to it, and the viscosity of the resultant mixture was controlled with ethyl acetate added thereto. This was processed in the same manner as in Example 1 to obtain a selegiline-containing percutaneous absorption-type pharmaceutical preparation.

EXAMPLE 3:

45 parts of the acrylic adhesive A, 40 parts of isopropyl myristate and 10 parts of selegiline were mixed and stirred in a container to give a uniform mixture. Next, 5 parts of sodium chloride dispersed in ethanol was added to the resultant acrylic adhesive A solution, and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 1 to obtain a selegiline-containing percutaneous absorption-type pharmaceutical preparation.

EXAMPLE 4:

47 parts of the acrylic adhesive B, 40 parts of isopropyl myristate and 10 parts of selegiline were mixed and stirred in a container to give a uniform mixture. Next, 3 parts of sodium chloride dispersed in ethanol was added to the resultant acrylic adhesive B solution, and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 1 to obtain a selegiline-containing percutaneous absorption-type pharmaceutical preparation.

COMPARATIVE EXAMPLE 1:

50 parts of the acrylic adhesive A, 40 parts of isopropyl myristate and 10 parts of selegiline were mixed and stirred in a container to give a uniform mixture. 0.3 parts (relative to the adhesive solid content) of (ethyl acetoacetate) aluminium diisopropylate was added to it, and the viscosityof the resultant mixture was controlled with ethyl acetate added thereto. This was processed in the same manner as in Example 1 to obtain a selegiline-containing percutaneous absorption-type pharmaceutical preparation.

COMPARATIVE EXAMPLE 2:

50 parts of the acrylic adhesive B, 40 parts of isopropyl myristate and 10 parts of selegiline were mixed and stirred in a container to give a uniform mixture. 0.3 parts (relative to the adhesive solid content) of (ethyl acetoacetate) aluminium diisopropylate was added to it, and the viscosityof the resultant mixture was controlled with ethyl acetate added thereto. This was processed in the same manner as in Example 1 to obtain a selegiline-containing percutaneous absorption-type pharmaceutical preparation.

COMPARATIVE EXAMPLE 3:

47 parts of the acrylic adhesive A and 40 parts of isopropyl myristate were mixed and stirred in a container to give a uniform mixture. In a different container, 10 parts of selegiline was mixed with stirring with 3 parts of glycerin (2-propanol solution controlled to 10 % by weight). Next, this was added to the previous acrylic adhesive A solution and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 1 to obtain a selegiline-containing percutaneous absorption-type pharmaceutical preparation.

COMPARATIVE EXAMPLE 4:

45 parts of the acrylic adhesive A and 40 parts of isopropyl myristate were mixed and stirred in a container to give a uniform mixture. In a different container, 10 parts of selegiline was mixed with stirring with 5 parts of glycerin (2-propanol solution controlled to 10 % by weight). Next, this was added to the previous acrylic adhesiveAsolution and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 1 to obtain a selegiline-containing percutaneous absorption-type pharmaceutical preparation.

COMPARATIVE EXAMPLE 5:

45 parts of the acrylic adhesive B and 40 parts of isopropyl myristate were mixed and stirred in a container to give a uniform mixture. In a different container, 10 parts of selegiline was mixed with stirring with 5 parts of glycerin (2-propanol solution controlled to 10 % by weight). Next, this was added to the previous acrylic adhesive B solution and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 1 to obtain a selegiline-containing percutaneous absorption-type pharmaceutical preparation.

(EXPERIMENTAL EXAMPLES)

The selegiline-containing percutaneous absorption-type pharmaceutical preparations produced in the above Examples and Comparative Examples were tested as in the gel fraction measurement test and the dipping test mentioned below.

EXPERIMENTAL EXAMPLE 1:

(Gel Fraction Measurement Test)

The proportion of the gel component insoluble in ethyl acetate, remaining in the preparations, was determined according to the method mentioned below.

The preparation was punched out to give 25-cm$^2$ pieces (5 cm x 5 cm). Two pieces were stuck to a porous tetrafluoroethylene film (20 cm x 10 cm) (substrate) of which the weight had been previously measured. This was folded so that the contents could not drop from it, and its weight was measured. This was put into a beaker. Two different types of solvents (type 1: ethyl acetate, type 2: ethyl acetate with 0.4 wt.% lactic acid) were separately added to the beakers so that the substrate could be completely immersed therein. On the next day, the solution in each beaker was removed, a solvent (ethyl acetate alone) was added to it. The solvent was exchanged everyday. After three exchanges, the solution in each beaker was removed, and the sample was dried, and its weight was measured. The gel fraction in the tested sample is calculated according to the following formula:

$$
\begin{aligned}
&\text{Gel Fraction (\%)} \\
&= 100 \times ((\text{sample weight after drying} - \text{substrate weight} - \text{support} \\
&\quad \text{weight}) - (\text{weight of sodium chloride in preparation})) / ((\text{sample} \\
&\quad \text{weight before drying} - \text{substrate weight} - \text{support weight}) \times \\
&\quad (\text{proportion of adhesive component in preparation}))
\end{aligned}
$$

EXPERIMENTAL EXAMPLE 2:

(Dipping Test)

On the assumption of actual application thereof to the skin, the cohesive force of the adhesive layer was determined according to the method mentioned below. The preparation was punched out to give 10-cm$^2$ pieces (3.16 cm x 3.16 cm). 5 ml of 0.4 wt.% lactic acid-containing physiological saline was put into a laboratory dish of glass, and the punched piece was, after its separator had been stripped off, dipped in the saline so that its adhesive side could face downward (the sample piece floated in the saline). After dipped for 24 hours, the sample piece was taken out, and its surface was dried. Panelists touched the dry surface with their fingers, and evaluated the tested samples. The results are given in Table 1.

Table 1

| | Gel Fraction (%) Type 1 | Gel Fraction (%) Type 2 | Evaluation before and after dipping |
|---|---|---|---|
| Example 1 | 83.2 | 52.0 | no cohesive failure |
| Example 2 | 86.2 | 55.6 | no cohesive failure |
| Example 3 | 89.6 | 61.0 | no cohesive failure |
| Example 4 | 85.2 | 51.8 | no cohesive failure |
| Comparative Example 1 | 81.0 | 24.8 | cohesive failure |

(continued)

|  | Gel Fraction (%) Type 1 | Gel Fraction (%) Type 2 | Evaluation before and after dipping |
|---|---|---|---|
| Comparative Example 2 | 88.1 | 10.7 | cohesive failure |
| Comparative Example 3 | 79.5 | 15.3 | cohesive failure |
| Comparative Example 4 | 82.1 | 16.1 | cohesive failure |
| Comparative Example 5 | 81.7 | 12.4 | cohesive failure |

The results in Table 1 confirm the following: The data of gel fraction in type 1 may indicate that the tested samples could seemingly keep their crosslinked structures. However, as in the test with type 2 in which lactic acid, a component of sweat, was added to ethyl acetate, on the assumption of actual application of the preparations to the skin, the samples of Examples 1 to 4 kept a value of more than 50 %, though lower than the value in the test with type 1. As understood from the test data before and after dipping, it has been confirmed that the adhesive layer does not suffer cohesive failure so far as it keeps the gel fraction level as in these Examples. In addition, it is also understood that, when the sodium chloride concentration in the preparations is increased, then the gel fraction level further increases. As opposed to these, it is understood that the value significantly lowered in Comparative Examples 1 and 2 where no sodium chloride was added to the preparations, and the samples suffered cohesive failure. In addition, it has been confirmed that the gel fraction value of the sample with glycerin also significantly lowered, like in these Comparative Examples, and the sample therefore suffered cohesive failure.

<Percutaneous Absorption-type Pharmaceutical Preparations Produced Using Selegiline Hydrochloride>

EXAMPLE 5:

45.85 parts of the acrylic adhesive A and 40 parts of isopropyl myristate were mixed and stirred in a container to give a uniform mixture. In a different container, 12 parts of selegiline hydrochloride was mixed with 2.15 parts of sodium hydroxide (10 % by weight) dissolved in ethanol, with stirring. Next, this was added to the previous acrylic adhesive A solution, and stirred. 0.3 parts (relative to the adhesive solid content) of (ethyl acetoacetate)aluminium diisopropylate was added to it, and the viscosity of the resultant mixture was controlled with ethyl acetate added thereto, and this was applied to a polyester film (75 $\mu$m thick) so that its dry thickness thereon could be 80 $\mu$m, and dried. This was stuck to a polyester film (12 $\mu$m thick), and aged at 70°C for 48 hours to obtain a selegiline hydrochloride-containing percutaneous absorption-type pharmaceutical preparation.

EXAMPLE 6:

38.8 parts of the acrylic adhesive A and 40 parts of isopropyl myristate were mixed and stirred in a container to give a uniform mixture. In a different container, 18 parts of selegiline hydrochloride was mixed with 3.20 parts of sodium hydroxide (10 % by weight) dissolved in ethanol, with stirring. Next, this was added to the previous acrylic adhesive A solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

EXAMPLE 7:

31.71 parts of the acrylic adhesive A and 40 parts of isopropyl myristate were mixed and stirred in a container to give a uniform mixture. In a different container, 24 parts of selegiline hydrochloride was mixed with 4.29 parts of sodium hydroxide (10 % by weight) dissolved in ethanol, with stirring. Next, this was added to the previous acrylic adhesive A solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

EXAMPLE 8:

45.85 parts of the acrylic adhesive B and 40 parts of isopropyl myristate were mixed and stirred in a container to give a uniform mixture. In a different container, 12 parts of selegiline hydrochloride was mixed with 2.15 parts of sodium hydroxide (10 % by weight) dissolved in ethanol, with stirring. Next, this was added to the previous acrylic adhesive B solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 6:

41.1 parts of the acrylic adhesive A and 40 parts of isopropyl myristate were mixed and stirred in a container to give a uniform mixture. In a different container, 12 parts of selegiline hydrochloride was mixed with stirring with 5 parts of diisopropanolamine and 1.9 parts of diethanolamine (both in 2-propanol solution controlled at 10 % by weight). Next, this was added to the previous acrylic adhesive A solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 7:

40.5 parts of the acrylic adhesive A and 40 parts of isopropyl myristate were mixed and stirred in a container to give a uniform mixture. In a different container, 12 parts of selegiline hydrochloride was mixed with stirring with 5 parts of diisopropanolamine and 2.5 parts of diethanolamine (both in 2-propanol solution controlled at 10 % by weight). Next, this was added to the previous acrylic adhesive A solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 8:

43.6 parts of the acrylic adhesive A and 40 parts of isopropyl myristate were mixed and stirred in a container to give a uniform mixture. In a different container, 12 parts of selegiline hydrochloride was mixed with stirring with 1. 9 parts of diethanolamine and 2.5 parts of monoethanolamine (both in 2-propanol solution controlled at 10 % by weight) . Next, this was added to the previous acrylic adhesive A solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 9:

41.1 parts of the acrylic adhesive B and 40 parts of isopropyl myristate were mixed and stirred in a container to give a uniform mixture. In a different container, 12 parts of selegiline hydrochloride was mixed with stirring with 5 parts of diisopropanolamine and 1.9 parts of diethanolamine (both in 2-propanol solution controlled at 10 % by weight) . Next, this was added to the previous acrylic adhesive B solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 10:

40.5 parts of the acrylic adhesive B and 40 parts of isopropyl myristate were mixed and stirred in a container to give a uniform mixture. In a different container, 12 parts of selegiline hydrochloride was mixed with stirring with 5 parts of diisopropanolamine and 2.5 parts of monoethanolamine (both in 2-propanol solution controlled at 10 % by weight). Next, this was added to the previous acrylic adhesive B solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 11:

43.6 parts of the acrylic adhesive B and 40 parts of isopropyl myristate were mixed and stirred in a container to give a uniform mixture. In a different container, 12 parts of selegiline hydrochloride was mixed with stirring with 1.9 parts of diethanolamine and 2.5 parts of monoethanolamine (both in 2-propanol solution controlled at 10 % by weight) . Next, this was added to the previous acrylic adhesive B solution, and stirred. 0.3 parts (relative to the adhesive solid content) of (ethyl acetoacetate)aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 12:

36.1 parts of the acrylic adhesive B and 40 parts of isopropyl myristate were mixed and stirred in a container to give a uniform mixture. In a different container, 12 parts of selegiline hydrochloride was mixed with stirring with 5 parts of diisopropanolamine, 1.9 parts of diethanolamine and 5 parts of glycerin (all in 2-propanol solution controlled at 10 % by weight) . Next, this was added to the previous acrylic adhesive B solution, and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 13:

35.5 parts of the acrylic adhesive A and 40 parts of isopropyl myristate were mixed and stirred in a container to give a uniform mixture. In a different container, 12 parts of selegiline hydrochloride was mixed with stirring with 5 parts of diisopropanolamine, 2.5 parts of monoethanolamine and 5 parts of glycerin (all in 2-propanol solution controlled at 10 % by weight) . Next, this was added to the previous acrylic adhesive A solution, and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

COMPARATIVE EXAMPLE 14:

38.6 parts of the acrylic adhesive A and 40 parts of isopropyl myristate were mixed and stirred in a container to give a uniform mixture. In a different container, 12 parts of selegiline hydrochloride was mixed with stirring with 1. 9 parts of diethanolamine, 2.5 parts of monoethanolamine and 5 parts of glycerin (all in 2-propanol solution controlled at 10 % by weight) . Next, this was added to the previous acrylic adhesive A solution, and stirred. 0.3 parts (relative to the adhesive solid content) of (ethyl acetoacetate) aluminium diisopropylate was added to it, and the viscosity of the resultant mixture was controlled with ethyl acetate added thereto. This was processed in the same manner as in Example 5 to obtain a selegiline hydrochloride-containing percutaneous absorption-type pharmaceutical preparation.

EXPERIMENTAL EXAMPLES:

The selegiline hydrochloride-containing percutaneous absorption-type pharmaceutical preparations produced in the above Examples and Comparative Examples were tested in the gel fraction measurement test as in Experimental Example 1 and in the dipping test as in Experimental Example 2. The results are given in Table 2.

Table 2

| | Gel Fraction (%) Type 1 | Gel Fraction (%) Type 2 | Evaluation before and after dipping |
|---|---|---|---|
| Example 5 | 86.6 | 54.6 | no cohesive failure |
| Example 6 | 89.5 | 52.7 | no cohesive failure |
| Example 7 | 96.8 | 59.8 | no cohesive failure |
| Example 8 | 85.5 | 58.6 | no cohesive failure |

(continued)

|  | Gel Fraction (%) Type 1 | Gel Fraction (%) Type 2 | Evaluation before and after dipping |
|---|---|---|---|
| Comparative Example 6 | 82.9 | 19.2 | cohesive failure |
| Comparative Example 7 | 85.2 | 15.0 | cohesive failure |
| Comparative Example 8 | 90.6 | 10.4 | cohesive failure |
| Comparative Example 9 | 65.4 | 30.2 | cohesive failure |
| Comparative Example 10 | 62.6 | 8.6 | cohesive failure |
| Comparative Example 11 | 59.2 | 23.6 | cohesive failure |
| Comparative Example 12 | 82.2 | 16.7 | cohesive failure |
| Comparative Example 13 | 79.6 | 11.5 | cohesive failure |
| Comparative Example 14 | 83.4 | 8.1 | cohesive failure |

The results in Table 2 confirm the following: The data of gel fraction in type 1 may indicate that the tested samples could seemingly keep their crosslinked structures. However, as in the test with type 2 in which lactic acid, a component of sweat, was added to ethyl acetate, on the assumption of actual application of the preparations to the skin, the samples of Examples 5 to 8 kept a value of more than 50 %, though lower than the value in the test with type 1. As understood from the test data before and after dipping, it has been confirmed that the adhesive layer does not suffer cohesive failure so far as it keeps the gel fraction level as in these Examples.

Even when the selegiline hydrochloride concentration therein was increased, the preparations of these Examples still kept the condition, not suffering cohesive failure. As opposed to these, it has been confirmed that, even in the comparative preparations in which alcohvlamine and glycerin were added in place of sodium hydroxide up to their uppermost limits, the gel fraction in the test with type 2 did not increase, or that is, the additives in these comparative preparations were ineffective.

## Claims

1. A percutaneous absorption-type pharmaceutical preparation which comprises: a support; and an adhesive layer containing a metal chloride, an adhesive and at least one of (-)-(R)-N,$\alpha$-dimethyl-N-2-propinylphenethylamine (Selegiline) and its hydrochloride, wherein the adhesive layer is subjected to a crosslinking treatment.

2. The percutaneous absorption-type pharmaceutical preparation of claim 1, wherein the crosslinking treatment is performed by a metal chelate compound.

3. The percutaneous absorption-type pharmaceutical preparation of claim 1, wherein the adhesive includes an acrylic polymer adhesive.

4. The percutaneous absorption-type pharmaceutical preparation of claim 1, wherein the adhesive layer contains a liquid plasticizer.

5. The percutaneous absorption-type pharmaceutical preparation of claim 4, wherein the liquid plasticizer is a fatty acid ester of a higher fatty acid having from 12 to 16 carbon atoms and a lower monoalcohol having from 1 to 4 carbon atoms.

## Patentansprüche

1. Pharmazeutische Zubereitung zur perkutanen Aufnahme, welche umfasst: einen Träger; und eine Klebstoffschicht, die ein Metallchlorid, einen Klebstoff und wenigstens eines von (-)-(R)-N,$\alpha$-Dimethyl-N-2-propinylphenethylamin (Selegilin) und seinem Hydrochlorid enthält, wobei die Klebstoffschicht einer Vernetzungsbehandlung unterzogen ist.

2. Pharmazeutische Zubereitung zur perkutanen Aufnahme nach Anspruch 1, wobei die Vernetzungsbehandlung durch

eine Metallchelatverbindung erfolgt.

**3.** Pharmazeutische Zubereitung zur perkutanen Aufnahme nach Anspruch 1, wobei der Klebstoff einen Acrylpolymerklebstoff einschließt.

**4.** Pharmazeutische Zubereitung zur perkutanen Aufnahme nach Anspruch 1, wobei die Klebstoffschicht einen flüssigen Weichmacher enthält.

**5.** Pharmazeutische Zubereitung zur perkutanen Aufnahme nach Anspruch 4, wobei der flüssige Weichmacher ein Fettsäureester von einer höheren Fettsäure mit 12 bis 16 Kohlenstoffatomen und einem niederen Monoalkohol mit 1 bis 4 Kohlenstoffatomen ist.


**Revendications**

**1.** Préparation pharmaceutique du type à absorption percutanée qui comprend : un support ; et une couche adhésive contenant un chlorure métallique, un adhésif et au moins l'un de (-)-(R)-N,$\alpha$-diméthyl-N-2-propynylphénéthylamine (sélegiline) et de son chlorhydrate, où la couche adhésive est soumise à un traitement de réticulation.

**2.** Préparation pharmaceutique du type à absorption percutanée selon la revendication 1 où le traitement de réticulation est accompli par un composé chélate métallique.

**3.** Préparation pharmaceutique du type à absorption percutanée selon la revendication 1 où l'adhésif inclut un adhésif polymère acrylique.

**4.** Préparation pharmaceutique du type à absorption percutanée selon la revendication 1 où la couche adhésive contient un plastifiant liquide.

**5.** Préparation pharmaceutique du type à absorption percutanée selon la revendication 4 où le plastifiant liquide est un ester d'acide gras d'un acide gras supérieur ayant de 12 à 16 atomes de carbone et d'un monoalcool inférieur ayant de 1 à 4 atomes de carbone.

**EP 1 731 143 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003062058 A
- JP 2004010525 A